(19) Europäisches Patentamt European Patent Office Office européen des brevets

(11) **EP 2 538 983 B1**

(12) **FASCICULE DE BREVET EUROPEEN**

(45) Date de publication et mention de la délivrance du brevet:
**29.07.2020 Bulletin 2020/31**

(51) Int Cl.:
*A61L 2/18* *(2006.01)*    *B08B 9/00* *(2006.01)*
*A23L 3/00* *(2006.01)*

(21) Numéro de dépôt: **11705936.0**

(22) Date de dépôt: **27.01.2011**

(86) Numéro de dépôt international:
**PCT/FR2011/050158**

(87) Numéro de publication internationale:
**WO 2011/104452 (01.09.2011 Gazette 2011/35)**

(54) **PROCEDE D'INERTAGE DE TANKS ASEPTIQUES**

VERFAHREN ZUR INERTISIERUNG KEIMFREIER TANKS

METHOD FOR INERTING ASEPTIC TANKS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **25.02.2010 FR 1051343**

(43) Date de publication de la demande:
**02.01.2013 Bulletin 2013/01**

(73) Titulaire: **L'Air Liquide Société Anonyme pour l'Etude et l'Exploitation des Procédés Georges Claude 75007 Paris (FR)**

(72) Inventeurs:
• **CAMPO, Philippe**
  **F-78180 Montigny le Bretonneux (FR)**
• **BOUQUIN, Fabrice**
  **F-78280 Guyancourt (FR)**

(74) Mandataire: **Mellul-Bendelac, Sylvie Lisette L'Air Liquide Direction des Services De la Propriété Intellectuelle 75, Quai d'Orsay 75321 Paris Cedex 07 (FR)**

(56) Documents cités:
**WO-A1-99/23016    WO-A1-03/070024 US-A- 5 544 669**

Remarques:
Le dossier contient des informations techniques présentées postérieurement au dépôt de la demande et ne figurant pas dans le présent fascicule.

**Description**

La présente invention concerne le domaine des procédés d'inertage de tanks utilisés pour le stockage ou encore la préparation de produits, et s'intéresse tout particulièrement au cas des tanks dits « aseptiques ».

Le document WO03/070024 illustre ce domaine technique.

Plus généralement, dans de nombreuses industries, il est nécessaire de préparer ou de maintenir des produits stockés dans des tanks ou conteneurs, sous des atmosphères contrôlées à faible teneur résiduelle en oxygène. Cette nécessité peut résulter de contraintes variables selon les produits stockées, et notamment liées à des raisons de sécurité, à une volonté de préserver la qualité du produit, du fait d'une sensibilité des produits stockés à l'oxydation etc....

Ce maintien sous atmosphère à faible concentration en oxygène de tels stockages est communément appelé « inertage ».

L'inertage consiste alors à substituer un volume d'air présent dans le tank à inerter, par un volume d'un gaz considéré comme inerte dans les conditions d'utilisation considérées. Les gaz les plus souvent utilisés dans de telles opérations sont l'azote, l'argon, ou encore le dioxyde de carbone, ou leurs mélanges.

Parmi les techniques le plus couramment utilisées pour inerter de tels tanks et donc pour substituer l'air par le gaz inerte on trouve :

- La purge par effet piston. Cette technique consiste à introduire le gaz inerte à l'opposé de la sortie du gaz purgé (évacué). Cette technique est surtout utilisée sur des sections faibles, très rarement pour des tanks, ceci pour des contraintes évidentes de dispersion du gaz. La quantité de gaz inerte consommée est proche d'un volume de gaz par volume de gaz à purger.
- L'inertage par dilution. Cette technique est très utilisée, elle convient aux capacités de toutes tailles. Elle consiste à injecter le gaz inerte par un orifice et à procéder ainsi à la dilution successive de l'air présent dans le tank. La dilution est maintenue jusqu'à obtenir la concentration en oxygène résiduel souhaitée.

La quantité de gaz inerte consommée avec cette technique est proche de 3 volumes de gaz par volume de capacité à inerter, cela pour atteindre une concentration en oxygène résiduelle typiquement comprise entre 2 et 5%.

- L'inertage par compression détente. Cette technique est très rarement utilisée pour inerter un tank. En effet, cette technique impose soit de réaliser une dépression du tank pour éliminer une partie de l'air présent, soit de mettre le tank sous pression pour faciliter la dilution. Les tanks sont donc très rarement adaptés aux pressions de travail requises.

La quantité de gaz inerte consommée avec cette technique dépend du nombre de cycles utilisés. Elle est souvent comprise entre 1 et 2 volumes de gaz inerte par volume de tank.

Venons en alors maintenant au cas particulier des tanks dits « aseptiques » :

Certaines industries telles que l'industrie agroalimentaire ou pharmaceutique doivent (pour des raisons bien compréhensibles que nous n'expliciterons pas davantage ici), procéder à des lavages réguliers de leurs tanks dans le cadre de l'aseptisation de ces tanks.

Les lavages aseptiques consistent, dans la majorité des cas, à laver le tank avec une solution chaude alcaline, à une température voisine de 80°C. On procède ensuite immédiatement après, sans attendre le refroidissement du tank, au rinçage du tank avec de l'eau froide. Cette eau froide (10 à 25°C) est injectée par les mêmes éléments de projection (type boules de lavages) situés au sommet du tank et utilisés précédemment pour envoyer la solution chaude de lavage.

Ce cyclage thermique entraîne, on le conçoit, une dépression du tank, dépression qui aspire l'air extérieur.

Plus précisément, lors du lavage à chaud, l'atmosphère de la cuve du tank se remplit d'un mélange gazeux qui comprend le gaz présent initialement dans la cuve, donc soit de l'air soit un gaz inerte si la cuve avait été initialement inertée, et de la vapeur d'eau dont le pourcentage dépend de la température (on le visualisera mieux plus loin grâce à des courbes).

Ensuite lors du rinçage de la cuve à l'eau froide, la vapeur d'eau se condense ce qui entraîne soit une dépression si le tank est fermé, soit l'aspiration d'un gaz, à savoir de l'air si la cuve est ouverte à l'atmosphère.

Pour les tanks dont l'atmosphère est contrôlée (inertée), cette mise à l'air est souvent effectuée par l'ouverture d'un évent permettant l'admission d'air pendant la phase de rinçage, ce qui a pour conséquence d'effectuer une entrée d'un volume important d'air, qu'il est nécessaire d'éliminer ultérieurement par l'une des techniques conventionnelles d'inertage déjà évoquées plus haut.

L'utilisation des techniques conventionnelles d'inertage imposent :

- Un temps d'immobilisation de la cuve important pour descendre la concentration en oxygène à une valeur suffisamment basse.

- Une consommation de gaz importante.
- Une gestion de l'inertage hasardeuse sans une consommation excessive de gaz inerte, sauf à contrôler l'atmosphère par analyse de l'oxygène résiduel, ce qui représente une solution coûteuse, en particulier dans le cas où plusieurs cuves sont à inerter.

Dans l'ensemble de ce contexte, on conçoit, et c'est un des objectifs de la présente invention, qu'il serait avantageux de pouvoir disposer d'un nouveau procédé d'inertage de tanks aseptiques, permettant à un tank après son étape de lavage aseptique, de conserver une atmosphère à faible teneur résiduelle en oxygène, tout en permettant d'atteindre :

- un temps d'immobilisation de la cuve quasi nul.
- une consommation de gaz optimisée et correspondant sensiblement à la stœchiométrie.
- préférentiellement, une gestion de la qualité de l'inertage qui soit assurée automatiquement.

Comme on le verra plus en détail dans ce qui suit, le procédé selon la revendication 1 propose d'effectuer un traitement gazeux « flash » du tank, durant tout ou partie du rinçage, et à fort débit, à l'aide d'un gaz inerte.
Et pour cela, afin de mieux expliciter ce qui précède, selon un des modes de mise en œuvre d'un tel traitement gazeux selon l'invention :
on effectue une prédétermination du besoin en gaz inerte pour réaliser ce traitement gazeux, on peut envisager différentes manières selon l'invention d'effectuer cette prédétermination, et notamment l'approche suivante :

a) après un lavage à chaud du tank, durant son rinçage (donc durant son fonctionnement traditionnel selon l'art antérieur), on établit la courbe de descente en température de la cuve du tank qui se produit durant ce rinçage, et l'on déduit de cette courbe un besoin en gaz inerte pour effectuer le traitement flash, on illustrera ceci plus loin par des exemples très pratiques.
La courbe de suivi de la température du tank durant le rinçage dans son fonctionnement traditionnel permet notamment de déterminer :

i) La température initiale avant le début du rinçage ;
j) Le temps pendant lequel le besoin en gaz est le plus important (ceci correspond à la chute brutale de température) et la température associée ;
k) Le temps pour atteindre la température finale d'équilibre de la cuve (et la température associée).

b) mais l'on peut également établir ce besoin de façon moins précise, (mode non-revendiquée), plus grossière, mais tenant compte de l'expérience, d'après les principales indications de température de lavage, température de l'eau de rinçage et temps de rinçage caractéristiques du site utilisateur considéré, puis d'ajuster plus précisément le débit injecté au moment des premiers tests de mise en place .
c) enfin, on peut déterminer ce besoin en gaz et réguler le débit injecté selon un mode proportionnel à la température de la cuve (on détaillera ce mode plus loin dans la présente demande).

Ici encore on aura établi après un lavage à chaud du tank, durant son rinçage (donc durant son fonctionnement traditionnel selon l'art antérieur), la courbe de descente en température du tank qui se produit durant ce rinçage.
Pour résumer ce mode on peut dire que l'on considère par ailleurs la courbe de tension de vapeur d'eau en fonction de la température dans un espace clos, et qu'à chaque température de la cuve va correspondre un besoin en azote (volume V en $m^3$) pour supprimer la vapeur d'eau (comme si la température de la cuve était de 0°C). Une fois prédéterminé ce besoin en gaz inerte, par l'une ou l'autre de ces méthodes, on effectue une compensation de ce besoin en injectant un débit très important de gaz inerte (cette notion de débit « important » sera mieux explicitée plus loin), cette injection étant démarrée préférentiellement durant la période intermédiaire entre lavage et rinçage, l'injection pouvant se poursuivre ou non durant tout ou partie du rinçage proprement dit selon la consigne de température que l'on souhaite atteindre.
A titre illustratif : on démarre l'injection quelques secondes avant d'ouvrir la vanne d'arrivée de l'eau froide de rinçage, et l'on stoppe l'injection flash lorsque la température de la cuve a atteint une consigne donnée, par exemple 25°C, même si à ce stade le rinçage de la cuve à l'eau froide se poursuit.
On recommande en effet selon l'invention d'injecter le gaz préférentiellement durant tout le rinçage mais sinon au moins jusqu'à sensiblement la fin de la période de chute rapide de température que l'on visualisera mieux dans le cadre d'exemples plus loin ci-dessous.
Comme on vient de l'indiquer, on préfère selon l'invention démarrer l'injection avant le début du rinçage (avant l'ouverture de la vanne d'arrivée d'eau), mais on peut sans inconvénient démarrer cette injection quelques secondes après le début du rinçage (par exemple 1 à 10 seconde après) ce qui entraînera une faible entrée d'air qui, suivant le résultat que l'on souhaite atteindre en termes de résiduel d'oxygène, ne sera pas obligatoirement préjudiciable.

Il est à noter que l'on pourra avantageusement dans certains cas, maintenir, en particulier pour les tanks ouverts, un léger débit de gaz inerte afin d'éviter une pollution de l'atmosphère par une entrée d'air.

En effet, quand on stoppe l'injection flash, même si la température ne descend plus ou quasiment plus, la cuve peut rester plusieurs heures sans être utilisé. Dans ce cas, et en particulier si la cuve possède une communication permanente avec l'atmosphère par l'intermédiaire d'un évent de grande taille, il peut se produire une entrée d'air dans le temps, et une telle entrée est d'autant plus probable si la température continue de descendre.

Maintenir un léger débit de gaz, par exemple d'azote (par exemple 2 à 5 Nm3/h) sur une telle cuve ouverte évite cette entrée d'air.

On notera également qu'une telle entrée d'air peut également avoir lieu si l'on introduit un produit dans la cuve qui ne serait pas à l'équilibre avec l'atmosphère (ce qui est généralement le cas). En effet, le produit « pomperait » une partie du gaz de l'atmosphère, d'autant plus rapidement qu'il serait éloigné de l'équilibre et encore plus s'il est agité. L'atmosphère « pompée » entraînerait une entrée d'air. Ici encore le maintien d'un léger débit de gaz inerte apporte une solution à ce phénomène.

Comme il apparaîtra clairement à l'homme du métier, selon l'art antérieur, lors du rinçage de la cuve à l'eau froide, la vapeur d'eau se condense ce qui entraîne soit une dépression si le tank est fermé, soit l'aspiration d'un gaz, à savoir de l'air quand la cuve est ouverte à l'atmosphère. Et donc grâce à la mise en œuvre du procédé selon l'invention c'est un gaz inerte qui intervient, ce qui change tout.

La présente invention concerne alors un procédé de traitement de tanks utilisés pour le stockage et/ou la préparation de produits, conforme à la revendication 1 ci-après annexée.

On notera que le document WO03/070024 cité plus haut a choisi, lui, une mise en œuvre totalement différente de celle présentement proposée puisque ce document propose de remplir complètement le tank d'eau, puis ensuite seulement d'injecter le gaz inerte pour chasser l'eau hors du tank (on se reportera notamment au bas de la page 2 du document, et aux figures 2 et 3) .

Outre que cette solution est économiquement absurde du fait de la consommation d'eau induite et de son pompage, elle est différente de celle présentement revendiquée qui propose d'injecter le gaz dans le tank, durant tout ou partie de l'étape de rinçage, donc durant une phase d'injection d'eau de rinçage, ceci notamment pour profiter de la dépression générée par le refroidissement de la cuve et ainsi optimiser la consommation de gaz. Selon le mode A/ de mise en œuvre de la présente invention, on injecte le gaz durant tout ou partie de la section de la courbe présentant la chute la plus brutale de température et l'on détermine le débit de gaz inerte à injecter par la formule suivante:

$$Q_1 = (V1_{gaz} / t_1) * 60$$

en Nm$^3$/h de gaz inerte
Avec:

$Q_1$ = le Débit de gaz inerte à injecter durant la section de la courbe correspondant à la chute la plus brutale de température

$$V1_{gaz} = (Pi - P_1) * V_{cuve}$$

$V_{cuve}$ = le volume de la cuve du tank en m
$P_i$ = la pression de vapeur d'eau saturante à la température T de la cuve à l'issue du lavage (en bar)
$P_1$ = la pression de vapeur d'eau saturante à la température T de la cuve à l'issue de la section de la courbe correspondant à la chute la plus brutale de température (en bar)
$t_1$ = la durée de la section de la courbe correspondant à la chute la plus brutale de température.

Selon le mode B/ de mise en œuvre de la présente invention, on injecte le gaz durant tout ou partie de la courbe de descente en température de la cuve du tank se produisant durant le rinçage, et en ce que l'on détermine les débits de gaz inerte $Q_1$ et $Q_2$ à injecter par les formules suivantes :

$$Q_1 = (V1_{gaz} / t_1) * 60$$

en Nm$^3$/h de gaz inerte
Avec :

$Q_1$ = le Débit de gaz inerte à injecter durant la section de la courbe correspondant à la chute la plus brutale de température

$$V1_{gaz} = (P_i - P_1) * V_{cuve}$$

$V_{cuve}$ = le volume de la cuve du tank en $m^3$

$P_i$ = la pression de vapeur d'eau saturante à la température T de la cuve à l'issue du lavage (en bar)

$P_1$ = la pression de vapeur d'eau saturante à la température $T_1$ de la cuve à l'issue de la section de la courbe correspondant à la chute la plus brutale de température (en bar)

$t_1$ = la durée de la section de la courbe correspondant à la chute la plus brutale de température. et :

$$Q_2 = (V2_{gaz} / (t_f - t_1)) * 60 \P$$

en $Nm^3/h$ de gaz inerte)

$Q_2$ = le Débit de gaz inerte à injecter durant la section de la courbe correspondant à la chute la plus lente de température, qui suit la section de la courbe présentant la chute la plus brutale de température.

$$V2_{gaz} = (P_1 - P_2) * V_{cuve}$$

$V_{cuve}$ = le volume de la cuve en $m^3$

$P^1$ = la pression de vapeur d'eau saturante à la température $T_1$ de la cuve à l'issue de la section de la courbe correspondant à la chute la plus brutale de température (en bar)

$P_2$ = la pression de vapeur d'eau saturante à la température T correspondant à la fin de la section de la courbe correspondant à la chute la plus lente de température (en bar)

$t_1$ = la durée de la section de la courbe correspondant à la chute la plus brutale de température

$t_f$ = le temps, depuis le début du rinçage, pour atteindre la température finale d'équilibre.

La présente invention pourra par ailleurs adopter un mode de mise en œuvre selon l'une ou plusieurs des sous-revendications ci-après annexées.

Comme on l'illustrera mieux ci-dessous, les expérimentations effectuées par la Demanderesse démontrent clairement l'apport positif de l'invention.

On a enregistré la température d'une cuve au moment de son rinçage et refroidissement (après passage de la solution de lavage à 80 °C). Cette cuve sera celle utilisée pour les exemples, l'un comparatif et l'autre selon l'invention, rapportés plus loin dans la présente description.

Cette courbe de refroidissement montre la présence d'au moins deux périodes de temps :

- un refroidissement important (brutal) et donc une dépression associée équivalente,
- un refroidissement faible (plus lent et de faible amplitude) et une dépression associée équivalente.
- La température initiale avant le début du rinçage est Ti= 80°C
- Le temps pendant lequel la chute de température est la plus sévère, et donc durant lequel le besoin en gaz inerte est le plus important, ce temps est voisin de $t_1$=4 mn
- La température à l'issue de cette première période (de chute sévère) est d'environ $T_1$=30°C
- Le temps pour atteindre la température finale est voisin de $t_f$=7 mn
- La température finale à ce point est d'environ $T_f$=20°C

Comme on l'a déjà expliqué plus haut dans la présente description, le besoin en gaz inerte au cours de ce refroidissement correspond en majeure partie à la compensation de la différence de pression partielle de vapeur d'eau aux différentes températures.

Les expérimentations ont par ailleurs permis de déduire les données suivantes :

- la pression partielle de l'eau à la température initiale Ti= 80°C est voisine de Pi=0,474 bar
- la pression partielle de l'eau à la température $T_1$=30°C est voisine de $P_1$= 0,042 bar
- la pression partielle de l'eau à la température finale $T_f$=20°C est voisine de $P_2$=0,023 bar.

Le besoin en gaz inerte **$V_{gaz}$** correspond alors au Volume de la cuve **$V_{cuve}$** multiplié par la différence de pression partielle de vapeur d'eau entre chaque étape, comme détaillé ci-dessous. Pour l'étape de chute rapide de température, durant la période de temps $t_1$ = 4mn, le besoin est de :

$$V1_{\textbf{gaz}} = (\text{Pi} = 0,474 \text{ bar} - P_1 = 0,042 \text{ bar}) * V_{\textbf{cuve}}$$

Pour l'étape de chute lente de température, permettant d'atteindre la température finale d'équilibre de la cuve au temps $t_f$=7 mn, le besoin est de :

$$V2_{\textbf{gaz}} = (P_1 = 0,042 \text{ bar} - P_2 = 0,023 \text{ bar}) * V_{\textbf{cuve}}$$

Ainsi, pour une cuve de volume $V_{\textbf{cuve}}$ =75 m$^3$, les besoins aux différents étapes sont :

$$V1_{gaz} = (0,474 - 0,042) * 75 = 32,4 \text{ m}^\textbf{3}$$

de gaz et

$$V2_{gaz} = (0,042 - 0,023) * 75 = 1,4 \text{ m}^\textbf{3}$$

de gaz.

Ces besoins en gaz sont à injecter pendant tout ou partie des périodes de temps associées, c'est-à-dire :

- durant le temps $t_1$= 4mn, correspondant à la phase de descente rapide de température de la cuve,
- durant le temps $t_f$-$t_1$ (7-4) = 3mn, correspondant à la phase de descente lente de température de la cuve, jusqu'à son équilibre voisin de 20°C.

A ces volumes de gaz correspondent alors les débits de gaz inerte associés :

- pour la première période de temps $t_1$= 4mn, étape de chute rapide de température, le débit d'azote à injecter pour compenser la chute de température et préserver l'atmosphère de la cuve est de :

$$Q_1 = (V1_{gaz} / t1) * 60 = (32,4/4) *60 = 486 \text{ Nm}^3/\text{h}$$

de gaz inerte,
- pour la seconde période de temps $t_f$-$t_1$ (7-4) = 3mn, étape de chute lente de température, le débit d'azote à injecter pour compenser la chute de température et préserver l'atmosphère de la cuve est de :

$$Q_2 = (V2_{gaz} / (t_f - t_1) * 60 = (1,4/3) *60 = 28 \text{ Nm}^3/\text{h}$$

de gaz inerte.

On détaille dans ce qui suit les conditions d'exemples pratiques de mise en œuvre, l'un selon l'invention et l'autre comparatif.

Essai selon l'art antérieur :

Sur la cuve précédemment évaluée, d'un volume de 75 m$^3$, préalablement inertée et dont le résiduel d'oxygène dans la phase gazeuse est d'environ 2,5%, on procède à une séquence de lavage aseptique à l'aide d'une solution aseptique à 80 °C, sans précaution particulière. On procède immédiatement après à une étape de rinçage à froid, et l'on constate comme attendu une aspiration d'air par les vannes d'évent. Cette aspiration entraîne une modification importante du résiduel d'oxygène dans la phase gaz puisqu'à l'équilibre à 20°C, la teneur en oxygène dans la phase gazeuse est passée de 2,5% à 13% en 7 minutes ($t_f$). Comme il apparaîtra clairement à l'homme du métier, le fait d'avoir procédé à un pré-inertage de la cuve avant lavage permet de mieux démontrer la reprise d'air par la cuve du fait du cyclage thermique.

Essai avec mise en œuvre du procédé selon l'invention:

Sur la même cuve, ici encore préalablement inertée et dont le résiduel d'oxygène dans la phase gazeuse à l'issue du pré-inertage est d'environ 3,5% (la différence de résiduel d'oxygène avec le cas précédent est uniquement due à la difficulté expérimentale sur cette installation d'obtenir une valeur ajustée au 1% près), on procède ici encore à une séquence de lavage aseptique, à l'aide d'une solution aseptique à 80 °C. A la fin du lavage aseptique à chaud, quelques secondes (typiquement 5 secondes) avant que ne démarre le rinçage à froid, on met en œuvre une injection flash d'azote, selon le besoin en gaz inerte prédéterminé d'après les courbes de chutes de température de la cuve et de pression de vapeur d'eau.

Le besoin calculé (comme précédemment expliqué plus haut) fait apparaître un besoin d'injecter 492 $Nm^3$/h pendant les 4 premières minutes du lavage, suivi d'une injection de 24 $Nm^3$/h pendant les 3 minutes suivantes.

Des contraintes techniques nous obligent à injecter un débit très légèrement plus faible de 465 $Nm^3$/h.

Le résiduel d'oxygène dans la phase gaz à l'issue du rinçage est de 4,5%. Le procédé a donc permis de préserver l'atmosphère du tank pendant cette étape de refroidissement très rapide, avec une consommation de gaz proche de la stoechiométrie.

On a décrit dans ce qui précède un exemple détaillé de mise en œuvre de l'invention utilisant un calcul des quantités de gaz inerte à mettre en œuvre de façon différenciée durant la portion de chute brutale de la température de la cuve, et durant la portion de chute lente de la température de la cuve.

On décrit dans ce qui suit, un autre mode c/ de mise en œuvre de l'invention i.e. un autre mode de calcul du besoin en gaz et de régulation de l'injection d'azote.

Ce mode utilise l'approche suivante, que l'on va illustrer ici encore dans le cas de la cuve de 75 $m^3$ utilisée dans les exemples précédents, mode où l'on va réguler le débit injecté avec une vanne proportionnelle en fonction de la température de la cuve

- ici encore on a établi après un lavage à chaud du tank, durant son rinçage (donc durant son fonctionnement traditionnel selon l'art antérieur), la courbe de descente en température du tank qui se produit durant ce rinçage,
- on considère par ailleurs la courbe de tension de vapeur d'eau en fonction de la température dans un espace clos (les deux premières colonnes du tableau ci-dessous),
- on va associer à chaque température de la cuve un besoin en azote (volume V en $m^3$) qui correspond au fait de supprimer la vapeur d'eau (comme si la température de la cuve était de 0°C ou -1°C). Ce besoin (volume) d'azote est présent en colonne 3 du tableau.

Considérons un exemple numérique, une température de cuve à 70°C, le besoin en gaz s'évalue par l'équation :

$$(P_{70} - P_0) * 75 \text{ m}^3 = (0.312 - 0.006) * 75 \text{ m}^3 = 23 \text{ m}^3$$

(Valeur que l'on retrouve bien en colonne 3 en face de la température 70 °C),
- considérons par exemple l'application d'un débit durant le temps de 4 mn de la portion de courbe présentant la chute brutale de température, on donne alors en colonne 4 du tableau ci-dessous le débit à injecter, en face de chaque température, basé sur ce temps de 4 mn.

Ce débit correspond donc à une application de l'inertage jusqu'à ce que soit atteinte une pression de vapeur d'eau nulle.
- bien entendu comme on l'a déjà signalé, on peut envisager d'arrêter l'inertage au voisinage de 25°C, il suffit alors de soustraire à chaque débit le débit d'inertage prévu pour la température de 25°C, on obtient alors la colonne 5 du tableau ci-dessous.
- bien entendu ceci ne constitue qu'une illustration de ce mode de mise en œuvre de l'invention, utilisant le seul temps de 4 mn correspondant à la chute brutale de température, mais cette même approche pourrait être utilisée pour un second temps, permettant de traiter la seconde pente (lente) de température.

| °C | Bar | Besoin en Azote (m3) | Débit t=4mn | Débit Arrêt à 25°C |
|---|---|---|---|---|
| 100 | 1,013 | | | |
| 95 | 0,845 | | | |
| 90 | 0,701 | | | |
| 85 | 0,583 | | | |
| 80 | 0,474 | 36 | 533 | 497 |
| 75 | 0,385 | 29 | 433 | 397 |
| 70 | 0,312 | 23 | 351 | 315 |
| 65 | 0,25 | 19 | 281 | 245 |

(suite)

| °C | Bar | Besoin en Azote (m3) | Débit t=4mn | Débit Arrêt à 25°C |
|---|---|---|---|---|
| 60 | 0,199 | 15 | 224 | 188 |
| 55 | 0,157 | 12 | 177 | 141 |
| 50 | 0,123 | 9,2 | 138 | 102 |
| 45 | 0,096 | 7,2 | 108 | 72 |
| 40 | 0,074 | 5,6 | 83 | 47 |
| 35 | 0,056 | 4,2 | 63 | 27 |
| 30 | 0,042 | 3,2 | 47 | 11 |
| 25 | 0,032 | 2,4 | 36 | 0 |
| 20 | 0,023 | 1,7 | 26 | |
| 15 | 0,017 | 1,3 | 19 | |
| 10 | 0,012 | 0,9 | 14 | |
| 5 | 0,009 | 0,7 | 10 | |
| 0 | 0,006 | 0 | 7 | |

On a tout particulièrement illustré dans ce qui précède l'utilisation d'un gaz d'inertage qui était de l'azote, mais on conçoit sans difficulté que selon les contextes, selon les applications et produits stockés, on pourra utiliser d'autres gaz et mélanges gazeux tels Ar, $CO_2$, He etc.....et leurs mélanges.

## Revendications

1.  Procédé de traitement de tanks utilisés pour le stockage et/ou la préparation de produits, procédé comprenant une étape de lavage aseptique à l'aide d'une solution aseptique alcaline à chaud du tank, à une température voisine de 80°C, suivie d'une étape de rinçage à l'eau froide du tank, à une température située dans la gamme allant de 10°C à 25°C, le procédé **se caractérisant en ce que** l'on injecte dans le tank, durant tout ou partie de l'étape de rinçage, un gaz inerte, et **en ce que** l'on effectue au préalable une prédétermination du besoin en gaz inerte pour réaliser ce traitement gazeux, cette prédétermination étant effectuée de la façon suivante :

    a) après un lavage aseptique à chaud du tank, à ladite température voisine de 80°C, on procède à son rinçage à l'eau froide dans ladite gamme 10°C-25°C, et l'on mesure la courbe de descente en température de la cuve du tank se produisant durant ce rinçage,
    b) on déduit de cette courbe, à partir de la connaissance de la section de la courbe présentant la chute de température la plus brutale, et de la section de la courbe correspondant à la chute la plus lente de température, qui suit la section de la courbe présentant la chute la plus brutale de température, ledit besoin en gaz inerte pour effectuer ledit traitement,

    et **en ce que** l'on injecte le gaz selon l'un des modes A/ ou B/ ou C/ suivants :

    Mode A/ : on injecte le gaz durant tout ou partie de la section de la courbe présentant la chute la plus brutale de température et l'on détermine le débit de gaz inerte à injecter par la formule suivante :

$$Q_1 = (V1_{gaz} / t_1) * 60$$

    en $Nm^3/h$ de gaz inerte) Avec :

    $Q_1$ = le Débit de gaz inerte à injecter durant la section de la courbe correspondant à la chute la plus brutale de température
    $V1_{gaz}$ = $(P_i - P_1) * V_{cuve}$
    $V_{cuve}$ = le volume de la cuve du tank en $m^3$
    $P_i$ = la pression de vapeur d'eau saturante à la température Ti de la cuve à l'issue du lavage, en bar
    $P_1$= la pression de vapeur d'eau saturante à la température $T_1$ de la cuve à l'issue de la section de la courbe correspondant à la chute la plus brutale de température, en bar

8

$t_1$ = la durée de la section de la courbe correspondant à la chute la plus brutale de température.

Mode B/ : on injecte le gaz durant tout ou partie de la courbe de descente en température de la cuve du tank se produisant durant le rinçage, et **en ce que** l'on détermine les débits de gaz inerte $Q_1$ et $Q_2$ à injecter par les formules suivantes :

$$Q_1 = (V1_{gaz} / t_1) * 60$$

en Nm$^3$/h de gaz inerte
Avec :
$Q_1$ = le Débit de gaz inerte à injecter durant la section de la courbe correspondant à la chute la plus brutale de température

$$V1_{gaz} = (P_i - P_1) * V_{cuve}$$

$V_{cuve}$ = le volume de la cuve du tank en m$^3$
$P_i$ = la pression de vapeur d'eau saturante à la température Ti de la cuve à l'issue du lavage, en bar
$P_1$ = la pression de vapeur d'eau saturante à la température $T_1$ de la cuve à l'issue de la section de la courbe correspondant à la chute la plus brutale de température, en bar
$t_1$ = la durée de la section de la courbe correspondant à la chute la plus brutale de température.

et :

$$Q_2 = (V2_{gaz} / (t_f - t_1)) * 60$$

en Nm$^3$/h de gaz inerte $Q_2$ = le Débit de gaz inerte à injecter durant la section de la courbe correspondant à la chute la plus lente de température, qui suit la section de la courbe présentant la chute la plus brutale de température.

$$V2_{gaz} = (P_1 - P_2) * V_{cuve}$$

$V_{cuve}$ = le volume de la cuve du tank en m$^3$
$P_1$ = la pression de vapeur d'eau saturante à la température $T_1$ de la cuve à l'issue de la section de la courbe correspondant à la chute la plus brutale de température, en bar
$P_2$ = la pression de vapeur d'eau saturante à la température $T_f$ correspondant à la fin de la section de la courbe correspondant à la chute la plus lente de température, en bar
$t_1$ = la durée de la section de la courbe correspondant à la chute la plus brutale de température
$t_f$ = le temps, depuis le début du rinçage, pour atteindre la température finale d'équilibre.

Mode C/ : on injecte le gaz en régulant le débit injecté avec une vanne proportionnelle en fonction de la température de la cuve, et l'on détermine le besoin en gaz inerte pour effectuer ledit traitement de la façon suivante :

- on associe à chaque température de la courbe de descente en température de la cuve un besoin en azote, exprimé comme un volume en m$^3$, correspondant au fait de supprimer la vapeur d'eau associée à cette température ;
- on associe à chaque besoin ainsi exprimé en volume en m$^3$ un débit de gaz, calculé pour un temps t de traitement.

2. Procédé de traitement de tanks selon la revendication 1, **se caractérisant en ce que** l'on démarre l'injection avant le début du rinçage.

3. Procédé de traitement de tanks selon la revendication 1, **se caractérisant en ce que** l'on démarre l'injection après le début du rinçage.

**4.** Procédé de traitement de tanks selon la revendication 2, **se caractérisant en ce que** l'on procède à l'injection du gaz inerte durant toute l'étape de rinçage.

**5.** Procédé de traitement de tanks selon la revendication 3, **se caractérisant en ce que** l'on procède à l'injection du gaz inerte durant tout le reste de l'étape de rinçage.

**6.** Procédé de traitement de tanks selon la revendication 2 ou 3, **se caractérisant en ce que** l'on arrête l'injection du gaz inerte lorsque la température de la cuve a atteint une consigne donnée.

**7.** Procédé de traitement de tanks selon la revendication 6, **se caractérisant en ce que** l'on arrête l'injection du gaz inerte lorsque la température de la cuve a atteint 30°C, préférentiellement lorsqu'elle a atteint 25 °C.

**8.** Procédé de traitement de tanks selon la revendication 2 ou 3, **se caractérisant en ce que** l'on arrête l'injection du gaz inerte lorsque la température de la cuve a atteint la fin de la section de la courbe correspondant à la chute la plus brutale de température.

**9.** Procédé de traitement de tanks selon l'une des revendications précédentes, **se caractérisant en ce que** l'on maintient, après l'arrêt de l'injection de gaz inerte, un débit de maintien de gaz inerte dans la cuve, débit de maintien qui est inférieur au débit de gaz injecté durant le traitement proprement dit.

**10.** Procédé de traitement de tanks selon le mode C/ de la revendication 1, **se caractérisant en ce que** le temps t de traitement est la durée de la section de la courbe présentant la chute de température la plus brutale.


**Patentansprüche**

**1.** Verfahren zur Behandlung von Tanks, die zum Lagern und/ oder Herstellen von Produkten verwendet werden, wobei das Verfahren einen Schritt zum keimfreien heißen Waschen des Tanks mithilfe einer keimfreien alkalischen Lösung bei einer Temperatur nahe 80 °C, gefolgt von einem Spülschritt des Tanks mit kaltem Wasser bei einer Temperatur umfasst, die sich in dem Bereich befindet, der von 10 °C bis 25 °C reicht, wobei das Verfahren **dadurch gekennzeichnet ist, dass** während des gesamten Spülschrittes oder eines Teils davon ein inertes Gas in den Tank eingespritzt wird, und dadurch, dass vorab eine Vorbestimmung des Bedarfs an inertem Gas zum Realisieren dieser Gasbehandlung durchgeführt wird, wobei diese Vorbestimmung wie folgt durchgeführt wird:

a) nach einem keimfreien heißen Waschen des Tanks bei der Temperatur nahe 80 °C wird dessen Spülung mit kaltem Wasser in dem Bereich, der von 10 °C bis 25 °C reicht, vorgenommen, und die Kurve des Temperaturabfalls im Sumpf des Tanks, der während des Spülens stattfindet, gemessen,
b) aus dieser Kurve wird ausgehend von der Kenntnis des Abschnitts der Kurve, der den brutalsten Temperatursturz aufweist, und des Abschnitts der Kurve, der dem langsamsten Temperatursturz entspricht, der auf den Abschnitt der Kurve folgt, der den brutalsten Temperatursturz aufweist, der Bedarf an inertem Gas zur Durchführung der Behandlung abgeleitet,

und dadurch, dass das Gas gemäß einem der folgenden Modi A/ oder B/ oder C/ eingespritzt wird:

Modus A/: das Gas wird während des gesamten Abschnitts der Kurve oder eines Teils davon, der den brutalsten Temperatursturz aufweist, eingespritzt, und der Durchsatz an einzuspritzendem inertem Gas wird durch die folgende Formel bestimmt:

$$Q_1 = (V1_{Gas} / t_1) * 60$$

in ($Nm^3$/h an inertem Gas) mit:
$Q_1$ = der Durchsatz an einzuspritzendem inertem Gas während des Abschnitts der Kurve, der dem brutalsten Temperatursturz entspricht,

$$V1_{Gas} = (P_i - P_1) * V_{Sumpf}$$

$V_{Sumpf}$ = das Volumen des Sumpfes des Tanks in $m^3$
$P_i$ = Wasserdampfsättigungsdruck bei der Temperatur $T_i$ des Sumpfes am Ende des Waschens in bar
$P_1$ = Wasserdampfsättigungsdruck bei der Temperatur $T_1$ des Sumpfes am Ende des Abschnitts der Kurve, der dem brutalsten Temperatursturz entspricht, in bar
$t_1$ = die Dauer des Abschnitts der Kurve, der dem brutalsten Temperatursturz entspricht.

Modus B/: das Gas wird während des gesamten Abschnitts der Kurve des Temperaturabfalls im Sumpf des Tanks, der während des Spülens stattfindet, oder eines Teils davon eingespritzt, und dadurch, dass die Durchsätze an einzuspritzendem inertem Gas $Q_1$ und $Q_2$ durch die folgenden Formeln bestimmt werden:

$$Q_1 = (V1_{Gas} / t_1) * 60$$

in ($Nm^3$/h an inertem Gas) mit:
$Q_1$ = der Durchsatz an einzuspritzendem inertem Gas während des Abschnitts der Kurve, der dem brutalsten Temperatursturz entspricht,

$$V1_{Gas} = (P_i - P_1) * V_{Sumpf}$$

$V_{Sumpf}$ = das Volumen des Sumpfes des Tanks in $m^3$
$P_i$ = Wasserdampfsättigungsdruck bei der Temperatur $T_i$ des Sumpfes am Ende des Waschens in bar
$P_1$ = Wasserdampfsättigungsdruck bei der Temperatur $T_1$ des Sumpfes am Ende des Abschnitts der Kurve, der dem brutalsten Temperatursturz entspricht, in bar
$t_1$ = die Dauer des Abschnitts der Kurve, der dem brutalsten Temperatursturz entspricht.

und:

$$Q_2 = (V2_{Gas} / (t_f - t_1)) * 60$$

in ($Nm^3$/h an inertem Gas)
$Q_2$ = der Durchsatz an einzuspritzendem inertem Gas während des Abschnitts der Kurve, der dem langsamsten Temperatursturz entspricht, der auf den Abschnitt der Kurve folgt, der den brutalsten Temperatursturz aufweist.

$$V2_{Gas} = (P_1 - P_2) * V_{Sumpf}$$

$V_{Sumpf}$ = das Volumen des Sumpfes des Tanks in $m^3$
$P_1$ = Wasserdampfsättigungsdruck bei der Temperatur $T_1$ des Sumpfes am Ende des Abschnitts der Kurve, der dem brutalsten Temperatursturz entspricht, in bar
$P_2$ = Wasserdampfsättigungsdruck bei der Temperatur $T_f$, entsprechend dem Ende des Abschnitts der Kurve, der dem langsamsten Temperatursturz entspricht, in bar
$t_1$ = die Dauer des Abschnitts der Kurve, der dem brutalsten Temperatursturz entspricht
$t_f$ = die Zeit, vom Beginn des Spülens an, zum Erreichen der abschließenden Gleichgewichtstemperatur.

Modus C/: das Gas wird unter Regelung des eingespritzten Durchsatzes mit einem Proportionalventil in Abhängigkeit von der Temperatur des Sumpfes eingespritzt, und der Bedarf an inertem Gas zum Durchführen der Behandlung wird wie folgt bestimmt:

- jeder Temperatur in der Kurve des Temperaturabfalls im Sumpf wird ein Bedarf an Stickstoff zugewiesen, der als ein Volumen in $m^3$ ausgedrückt wird, entsprechend der Tatsache des Entfernens des dieser Temperatur zugewiesenen Wasserdampfes;
- jedem so in Volumen in $m^3$ ausgedrückten Bedarf wird ein Gasdurchsatz zugewiesen, der für eine Behandlungszeit t berechnet wird.

2. Verfahren zur Behandlung von Tanks nach Anspruch 1, **dadurch gekennzeichnet, dass** die Einspritzung vor Beginn des Spülens gestartet wird.

3. Verfahren zur Behandlung von Tanks nach Anspruch 1, **dadurch gekennzeichnet, dass** die Einspritzung nach Beginn des Spülens gestartet wird.

4. Verfahren zur Behandlung von Tanks nach Anspruch 2, **dadurch gekennzeichnet, dass** die Einspritzung des inerten Gases während des gesamten Spülschrittes vorgenommen wird.

5. Verfahren zur Behandlung von Tanks nach Anspruch 3, **dadurch gekennzeichnet, dass** die Einspritzung des inerten Gases während des gesamten Rests des Spülschrittes vorgenommen wird.

6. Verfahren zur Behandlung von Tanks nach Anspruch 2 oder 3, **dadurch gekennzeichnet, dass** die Einspritzung des inerten Gases gestoppt wird, wenn die Temperatur des Sumpfes einen gegebenen Sollwert erreicht hat.

7. Verfahren zur Behandlung von Tanks nach Anspruch 6, **dadurch gekennzeichnet, dass** die Einspritzung des inerten Gases gestoppt wird, wenn die Temperatur des Sumpfes 30 °C erreicht hat, vorzugsweise, wenn sie 25 °C erreicht hat.

8. Verfahren zur Behandlung von Tanks nach Anspruch 2 oder 3, **dadurch gekennzeichnet, dass** die Einspritzung des inerten Gases gestoppt wird, wenn die Temperatur des Sumpfes das Ende des Abschnitts der Kurve erreicht hat, die dem brutalsten Temperatursturz entspricht.

9. Verfahren zur Behandlung von Tanks nach einem der vorstehenden Ansprüche, **dadurch gekennzeichnet, dass** nach dem Stoppen der Einspritzung des inerten Gases ein Haltedurchsatz für inertes Gas in dem Sumpf beibehalten wird, wobei der Haltedurchsatz kleiner als der Durchsatz eines während der eigentlichen Behandlung eingespritzten Gases ist.

10. Verfahren zur Behandlung von Tanks gemäß dem Modus C/ des Anspruchs 1, **dadurch gekennzeichnet, dass** die Zeit t der Behandlung die Dauer des Abschnitts der Kurve ist, der den brutalsten Temperatursturz aufweist.

**Claims**

1. Method for treating tanks used for the storage and/or the preparation of products, method comprising an aseptic washing step using a hot alkaline aseptic solution of the tank, at a temperature close to 80°C, followed by a step of rinsing with cold water of the tank, at a temperature situated in the range going from 10°C to 25°C, the method being **characterised in that** an inert gas is injected into the tank, during all or some of the rinsing step, and **in that** a predetermination of the need for inert gas to carry out this gaseous treatment is carried out beforehand, this predetermination being carried out as follows:

a) after a hot aseptic washing of the tank, at said temperature close to 80°C, it is rinsed with cold water in said range 10°C - 25°C, and the temperature descent curve of the container of the tank occurring during this rinsing is measured,
b) from this curve, from the knowledge of the section of the curve presenting the most sudden fall in temperature, and of the section of the curve corresponding to the slowest fall in temperature, which follows the section of the curve presenting the most sudden fall in temperature, said need for inert gas to carry out said treatment is deduced,

and **in that** the gas is injected according to one of the following methods A/ or B/ or C/:

Method A/: the gas is injected during all or some of the section of the curve presenting the most sudden fall in temperature and the inert gas flow to be injected is determined by the following formula:

$$Q_1 = (V1_{gas} / t_1) \times 60$$

(in $Nm^3/h$ of inert gas)
With:
$Q_1$ = the inert gas flow to be injected during the section of the curve corresponding to the most sudden fall in temperature

$$V1_{gas} = (P_i - P_1) \times V_{container}$$

$V_{container}$ = the volume of the container of the tank in $m^3$
$P_i$ = the saturating steam pressure at the temperature Ti of the container after the washing, in bars
$P_1$ = the saturating steam pressure at the temperature $T_1$ of the container after the section of the curve corresponding to the most sudden fall in temperature, in bars
$t_1$ = the duration of the section of the curve corresponding to the most sudden fall in temperature.

Method B/: the gas is injected during all or some of the temperature descent curve of the container of the tank occurring during the rinsing, and **in that** the inert gas flows $Q_1$ and $Q_2$ to be injected are determined by the following formulas:

$$Q_1 = (V1_{gas} / t_1) \times 60$$

(in $Nm^3/h$ of inert gas)
With:
$Q_1$ = the inert gas flow to be injected during the section of the curve corresponding to the most sudden fall in temperature

$$V1_{gas} = (P_i - P_1) \times V_{container}$$

$V_{container}$ = the volume of the container of the tank in $m^3$
$P_i$ = the saturating steam pressure at the temperature Ti of the container after the washing, in bars
$P_1$ = the saturating steam pressure at the temperature $T_1$ of the container after the section of the curve corresponding to the most sudden fall in temperature, in bars
$t_1$ = the duration of the section of the curve corresponding to the most sudden fall in temperature.

and:

$$Q_2 = (V2_{gas} / (t_f - t_1) \times 60$$

(in $Nm^3/h$ of inert gas)
$Q_2$ = the inert gas flow to be injected during the section of the curve corresponding to the slowest fall in temperature, which follows the section of the curve presenting the most sudden fall in temperature.

$$V2_{gas} = (P_1 - P_2) \times V_{container}$$

$V_{container}$ = the volume of the container of the tank in $m^3$
$P_1$ = the saturating steam pressure at the temperature $T_1$ of the container after the section of the curve corresponding to the most sudden fall in temperature, in bars
$P_2$ = the saturating steam pressure at the temperature $T_f$ corresponding to the end of the section of the curve corresponding to the slowest fall in temperature, in bars
$t_1$ = the duration of the section of the curve corresponding to the most sudden fall in temperature,
$t_f$ = the time, from the start of the rinsing, to reach the final equilibrium temperature.

Method C/: the gas is injected by regulating the flow injected with a proportional valve according to the temperature of the container, and the need for inert gas is determined to carry out said treatment as follows:

- a need for nitrogen is associated with each temperature of the temperature descent curve of the container, expressed as a volume in $m^3$, corresponding to the action of removing the steam associated with this temperature;
- a gas flow is associated with each need thus expressed as a volume in $m^3$, calculated for a treatment time t.

2. Method for treating tanks according to claim 1, being **characterised in that** the injection is started before the start of the rinsing.

3. Method for treating tanks according to claim 1, being **characterised in that** the injection is started after the start of the rinsing.

4. Method for treating tanks according to claim 2, being **characterised in that** the injection of the inert gas is proceeded with during the whole rinsing step.

5. Method for treating tanks according to claim 3, being **characterised in that** the injection of the inert gas is proceeded with during all the remainder of the rinsing step.

6. Method for treating tanks according to claim 2 or 3, being **characterised in that** the injection of the inert gas is stopped when the temperature of the container has reached a given setpoint.

7. Method for treating tanks according to claim 6, being **characterised in that** the injection of the inert gas is stopped when the temperature of the container has reached 30°C, preferably when it has reached 25°C.

8. Method for treating tanks according to claim 2 or 3, being **characterised in that** the injection of the inert gas is stopped when the temperature of the container has reached the end of the section of the curve corresponding to the most sudden fall in temperature.

9. Method for treating tanks according to one of the preceding claims, being **characterised in that** a flow for maintaining inert gas in the container is maintained, after the injection of inert gas is stopped, a maintaining flow which is less than the gas flow injected during the treatment, strictly speaking.

10. Method for treating tanks according to method C/ of claim 1, being **characterised in that** the treatment time t is the duration of the section of the curve presenting the most sudden fall in temperature.

**EP 2 538 983 B1**

**RÉFÉRENCES CITÉES DANS LA DESCRIPTION**

**Documents brevets cités dans la description**

- WO 03070024 A